# EUROPEAN PATENT SPECIFICATION

(11) **EP 3 120 853 B1**
(45) Date of publication and mention of the grant of the patent: **22.09.2021**
(21) Application number: 15764825.4
(22) Date of filing: 20.03.2015
(51) Int. Cl.: A61K 31/558, A61K 48/00, A61K 31/704, A61P 35/00, A61K 31/7048, A61K 31/7105, A61K 31/713, C12N 15/113

(54) **PHARMACEUTICAL COMPOSITION FOR TREATING STK11-MUTATION CANCER USING CARDIAC GLYCOSIDES**
PHARMAZEUTISCHE ZUSAMMENSETZUNG ZUR BEHANDLUNG VON STK11-MUTATIONSKREBS MIT HERZGLYCOSIDEN
COMPOSITION PHARMACEUTIQUE POUR LE TRAITEMENT DE CANCER À MUTATION STK11 AU MOYEN DE GLYCOSIDES CARDIAQUES

(30) Priority: 20.03.2014 KR 20140032585; 19.03.2015 KR 20150038485
(43) Date of publication of application: 25.01.2017
(73) Proprietor: Industry-Academic Cooperation Foundation, Sookmyung Women's University, Seoul 140-742 (KR)
(72) Inventor: YOON, Suk Joon, Seoul 135-795 (KR); HE, Ning Ning, Seoul 140-132 (KR); KIM, Na Young, Seoul 152-841 (KR); CHO, Yong-Yeon, Seoul 152-821 (KR)
(74) Representative: Ter Meer Steinmeister & Partner
(86) International application number: PCT/KR2015/002755
(87) International publication number: WO 2015/142117

(56) References cited:
- WO-A1-2009/064657
- WO-A1-2013/012997
- US-A1- 2010 068 198
- US-A1- 2010 068 198
- Fiona Cahill ET AL: "Exploiting vulnerabilities in STK11 mutant cells to improve therapeutic responses in non small cell lung cancer (NSCLC)", , 6 November 2013 (2013-11-06), XP055410047, Retrieved from the Internet: URL:https://conference.ncri.org.uk/abstrac ts/2013/abstracts/B196.htm [retrieved on 2017-09-26]
- SHACKELFORD DAVID B ET AL: "LKB1 Inactivation Dictates Therapeutic Response of Non-Small Cell Lung Cancer to the Metabolism Drug Phenformin", CANCER CELL, CELL PRESS, US, vol. 23, no. 2, 24 January 2013 (2013-01-24), pages 143-158, XP028976809, ISSN: 1535-6108, DOI: 10.1016/J.CCR.2012.12.008
- BOUBACAR BENZIANE ET AL: "Activation of AMP-activated Protein Kinase Stimulates Na + ,K + -ATPase Activity in Skeletal Muscle Cells", JOURNAL OF BIOLOGICAL CHEMISTRY, vol. 287, no. 28, 6 July 2012 (2012-07-06) , pages 23451-23463, XP055409744, US ISSN: 0021-9258, DOI: 10.1074/jbc.M111.331926
- VARISA PONGRAKHANANON ET AL: "Ouabain Suppresses the Migratory Behavior of Lung Cancer Cells", PLOS ONE, vol. 8, no. 7, 13 October 2013 (2013-10-13), page e68623, XP055409513, DOI: 10.1371/journal.pone.0068623
- J P KOIVUNEN ET AL: "Mutations in the LKB1 tumour suppressor are frequently detected in tumours from Caucasian but not Asian lung cancer patients", BRITISH JOURNAL OF CANCER, vol. 99, no. 2, 1 July 2008 (2008-07-01), pages 245-252, XP055409528, GB ISSN: 0007-0920, DOI: 10.1038/sj.bjc.6604469
- NAYOUNG KIM ET AL: "Cardiac glycosides display selective efficacy for STK11 mutant lung cancer", SCIENTIFIC REPORTS, vol. 6, no. 1, 19 July 2016 (2016-07-19), XP055409515, DOI: 10.1038/srep29721
- CALDERON-MONTANO, J, M. ET AL.: 'The cardiac glycosides digitoxin, digoxin and ouabain induce a potent inhibition of glycolysis in lung cancer cells' THESIS NUMBER WMC004323 2013, pages 1 - 12, XP055225361
- JI, H. ET AL.: 'LKB1 modulates lung cancer differentiation and metastasis' NATURE vol. 448, no. 7155, 2007, pages 807 - 810, XP002496527
- DATABASE NCBI [Online] 10 October 2007 XP055225366 Database accession no. ABW03450.1
- ZHOU GAOCHAO ET AL: "Role of AMP-activated protein kinase in mechanism of metformin action", THE JOURNAL OF CLINICAL INVESTIGATION, B M J GROUP, GB, vol. 108, no. 8, 15 October 2001 (2001-10-15), pages 1167-1174, XP002604110, ISSN: 0021-9738, DOI: 10.1172/JCI200113505
- Yang Wanjuan ET AL: "Genomics of Drug Sensitivity in Cancer (GDSC): a resource for therapeutic biomarker discovery in cancer cells", Nucleic Acids Research, vol. 41, no. D1, 22 November 2012 (2012-11-22), pages D955-D961, XP055795266, ISSN: 0305-1048, DOI: 10.1093/nar/gks1111

## Description

### [Technical Field]

The present invention relates to a pharmaceutical composition for treating STK11-mutation cancer, which includes, as an active ingredient, a material for inhibiting a sodium-potassium transport function of Na⁺-K⁺ ATPase (ATP1A1), wherein the material comprises a cardiac glycoside selected from the group consisting of digoxin, oubain, and digitoxin.

The references to methods of treatment in this description are to be interpreted as references to the compounds, pharmaceutical compositions and medicaments of the present invention for use in a method for treatment of the human (or animal) body by therapy (or for diagnosis).

### [Background Art]

Lung cancer is a kind of cancer whose incidence rate has increased all over the world, and may be mainly classified into primary lung cancer in which cancer cells are first developed in the bronchi or pulmonary alveoli, and metastatic lung cancer in which cancer cells are developed in other organs and grow in the lungs by migrating through blood vessels or lymphatic vessels.

Lung cancer is mainly classified into non-small cell lung cancer (NSCLC) and small cell lung cancer (SCLC) in a histological aspect. Here, the non-small cell lung cancer amounts for 75% of the global distribution of lung cancer. The non-small cell lung cancer (NSCLC) is again classified into adenocarcinoma, squamous cell carcinoma, and large cell carcinoma. Among these, adenocarcinoma has been steadily developed at a higher frequency.

Although the incidence of lung cancer has rapidly increased due to an increase in the smoking population, air pollution, etc., most of the cases of lung cancer are difficult to treat using chemotherapy and radiotherapy. In cases of small cell carcinoma of small size, chemotherapy and radiotherapy may be applied to treat small cell carcinoma, but it is not possible to expect that chemotherapy and radiotherapy can completely cure small cell carcinoma. Non-small cell lung cancer cannot be treated only by chemotherapy since non-small cell lung cancer is less affected by an anticancer drug, compared to the small cell lung cancer. Therefore, surgical complete removal of a tumor is the only effective therapy. However, up to 30% of lung cancer patients have a tumor that cannot be completely removed at the time of diagnosis. Only a third or less of the lung cancer patients survive for 5 years after surgical resection. Therefore, there is an urgent demand for methods capable of more effectively treating lung cancer.

In addition, among the drugs recently approved by the US Food and Drug Administration (FDA), most cancer therapeutic drugs target a certain mutant type of carcinoma. Due to changes in the market for such anticancer drugs, recent development of cancer therapeutic agents has focused on searching for targets that can specifically control mutant carcinoma.

In particular, there are reports showing that STK11 (or LKB1) has a high mutation frequency in lung cancer and that the metastasis and differentiation of lung cancer are promoted in LKB1 mutant cells, as published in the journal Nature in 2007. As a result, it is known that STK11 plays an important role in lung cancer. In particular, STK11 mutation is mutually exclusive from other major lung cancer mutations in tissue samples from lung cancer patients, there is a need for the development of anti-cancer drugs specific to a group of STK11 mutant lung cancer patients. However, although clinical trials of anti-cancer drugs have been actively conducted on mutations of the genes such as ALK, BRAF, and EGFR, etc. in lung cancer, there is no report on the development of an anticancer drug that targets STK11-mutation cancer having the highest frequency of mutation.

The previous studies showed that the genetic variants in the STK11 signaling pathway may impact a therapeutic effect on diseases such as type II diabetes mellitus. STK11 codes for a serine-threonine kinase functioning as a tumor-inhibiting factor, and regulates cell polarity. The loss-of-function somatic mutation of STK11 has been found in approximately 30% of lung cancer, and has been proposed to promote metastasis.

Meanwhile, cardiac glycosides include a family of digoxin, digitoxin, and ouabain drugs, and have been used as conventional cardiotonic drugs to treat diseases such as congestive heart failure, and arrhythmia, etc. Cardiac glycosides inhibit a function of Na⁺-K⁺ ATPase (an ATP1A1 protein; ATPase, Na⁺-K⁺ transporting, alpha 1 polypeptide), and thus functions to regulate concentrations of intracellular sodium and potassium. The ATP1A1 protein functions to maintain a low concentration of sodium and a high concentration of potassium in the cells, and thus serves as an electrogenic pump that generates a potential difference between both sides of a cytoplasmic membrane.

Also, the ATP1A1 protein functions to determine whether excitable and contractile tissues are polarized. Here, depolarization and repolarization correspond to the influx of sodium and the release of potassium, respectively. Na⁺-K⁺ ATPase restores equilibrium. Further, the ATP1A1 protein functions to generate potential energy associated with ion gradients generated on both sides of a plasma membrane. This energy is especially used for auxiliary active transport, and thus is generally coupled to that of sodium.

Generally known cardiac glycosides bind to extracellular moieties, that is, potassium ions, of enzymes to transport the potassium ions into cells when the cardiac glycosides are in a phosphorylated state. The extracellular potassium ions which induce dephosphorylation of an alpha subunit reduce an effect of the cardiac glycosides. The cardiac glycosides inhibit a function of Na⁺-K⁺ ATPase (ATP1A1) in other tissues such as heart muscle, conducting tissue of the heart, smooth muscle, and red blood cells. They have little influence on Na⁺-K⁺ ATPase (ATP1A1) in skeletal muscle.

As the prior art associated with such ATP1A1 and cancer diseases, Korean Patent Application Publication No. 10-2013-0137562 (published on December 17, 2013) discloses a composition for diagnosing or treating lung cancer, which includes, as an active ingredient, a material specifically binding to at least one target polypeptide selected from the group consisting of apolipoprotein O (APOO), ATP1A1 (ATPase, Na⁺-K⁺ transporting, alpha 1 polypeptide), calnexin (CANX), and dolichyl-diphosphooligosaccharide-protein glycosyltransferase (DDOST). Specifically, the patent application discloses that ATP1A1 (ATPase, Na⁺-K⁺ transporting, alpha 1 polypeptide) is useful in diagnosing lung cancer. However, the patent application describes neither a specific therapeutic effect of the composition nor therapeutic efficacy against mutation cancer.

SHACKELFORD DAVID B ET AL: "LKB1 Inactivation Dictates Therapeutic Response of Non-Small Cell Lung Cancer to the Metabolism Drug Phenformin", CANCER CELL, CELL PRESS, 2013, vol. 23, no. 2, pages 143-158 suggests phenformin as a cancer-metabolism based therapeutic to selectively target LKB1-deficient tumors. LKB1 is also known as STK11.

CALDERON-MONTANO, J, M. ET AL.: "The cardiac glycosides digitoxin, digoxin and ouabain induce a potent inhibition of glycolysis in lung cancer cells", THESIS NUMBER WMC004323, 2013, pages 1-12 (https://www.webmedcentral.com/wmcpdf/Article_WMC004323.pdf) discloses that the cardiac glycosides digitoxin, digoxin and ouabain were more cytotoxic on A549 lung cancer cells than on MRC5 non-malignant lung fibroblasts.

### [Disclosure]

### [Technical Problem]

Therefore, it is an aspect of the present invention to provide a pharmaceutical composition having a specific therapeutic effect on cancers harboring STK11 mutation.

### [Technical Solution]

The present invention provides a pharmaceutical composition for use in treating cancers harboring STK11 (serine/threonine kinase 11) mutation, which includes, as an active ingredient, a material for inhibiting a sodium-potassium transport function of Na⁺-K⁺ ATPase (an ATP1A1 protein; sodium/potassium-transporting ATPase subunit alpha-1), wherein the material comprises a cardiac glycoside selected from the group consisting of digoxin, oubain, and digitoxin.

According to one exemplary embodiment of the present invention, the Na⁺-K⁺ ATPase may have an amino acid sequence set forth in SEQ ID NO: 1.

According to another exemplary embodiment of the present invention, the subject may be a mammal including a human, livestock, etc.

### [Advantageous Effects]

According to the present invention, the growth of lung cancers harboring STK11 mutations, which was the most frequently occurred among various cancer gene mutations detected in cancer cell lines, is inhibited when it is treated with cardiac glycosides selected from digoxin, oubain and digitoxin which have been used as a cardiotonic drug in the prior art.

### [Description of Drawings]

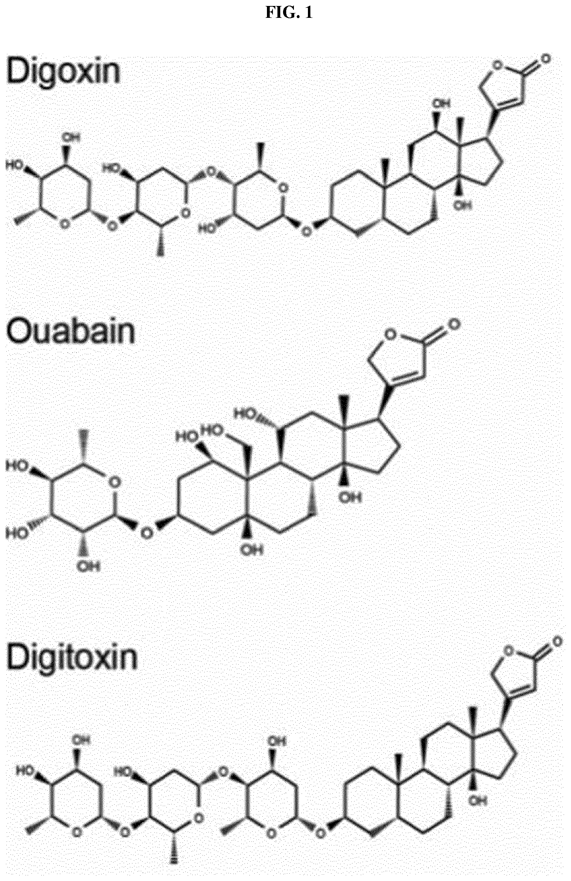

FIG. 1 shows structural formulas of cardiac glycosides proposed to be effective against STK11 mutant cancer according to one exemplary embodiment of the present invention.

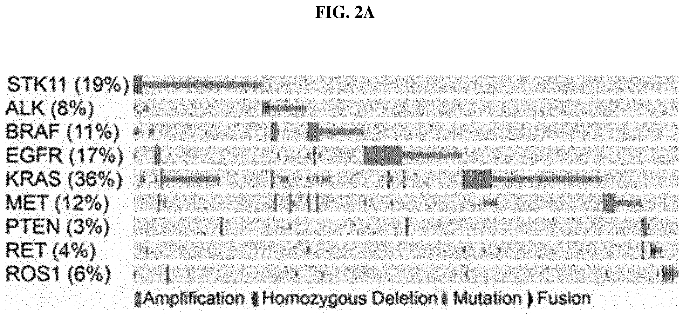

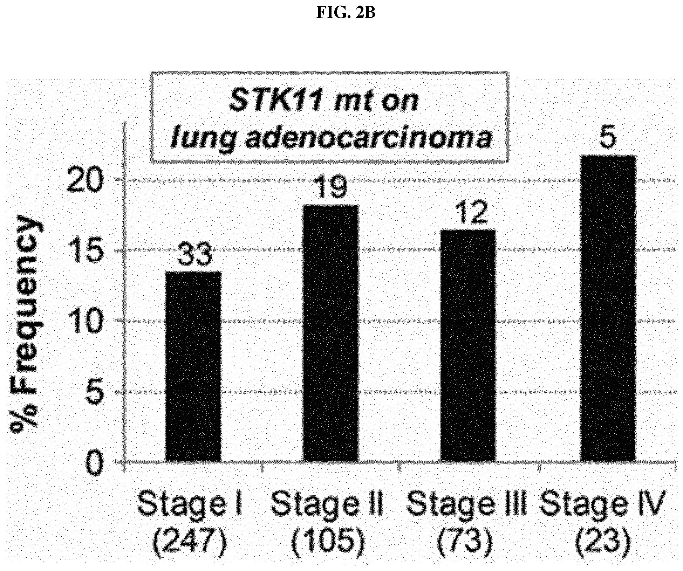

FIG. 2 shows results of determining STK11 mutation patterns in non-small cell lung cancer (NSCLC). FIG. 2A shows a genetic variation pattern of seven major mutations in patients suffering from lung adenocarcinoma which is one of NSCLC subtypes of the non-small cell lung cancer (NSCLC) and accounts for the highest proportion of non-small cell lung cancer (NSCLC). FIG. 2B shows STK11 mutation frequencies (%) according to the cancer progression of lung adenocarcinoma.

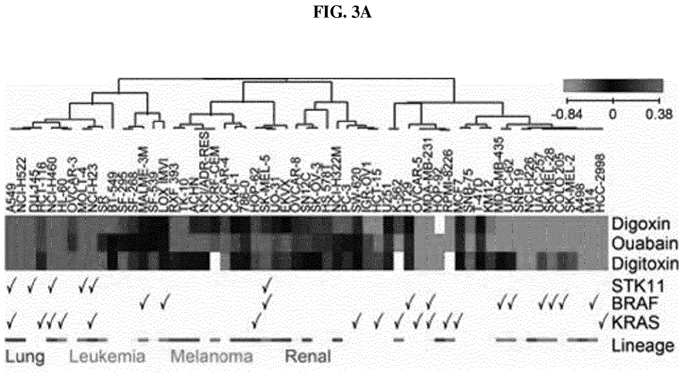

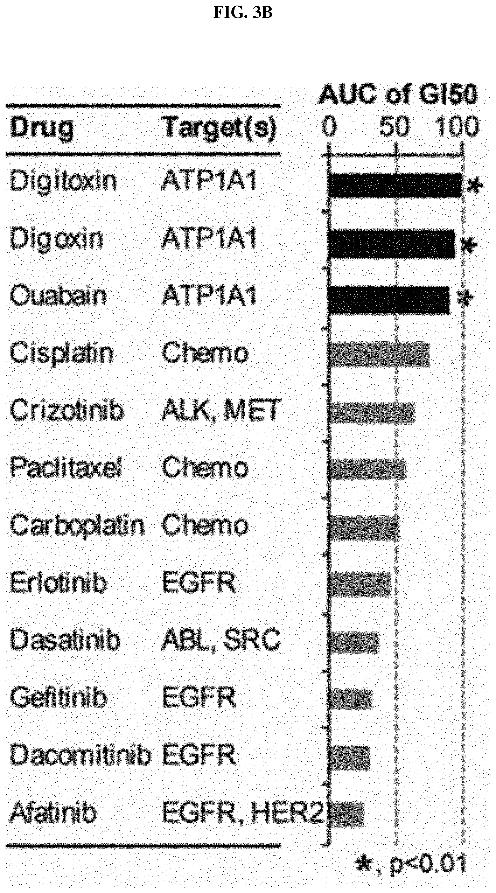

FIG. 3A is a heat map obtained by hierarchically clustering of the three cardiac glycosides shown in FIG. 1 in 60 cancer cell lines, and FIG. 3B shows results of measuring relative drug effects (AUC values at -logGI₅₀) of therapeutic agents for non-small cell lung cancer (NSCLC) used in the prior art and the cardiac glycosides in STK11 mutant cell lines, compared to those in cell lines in which STK11 is normally expressed.

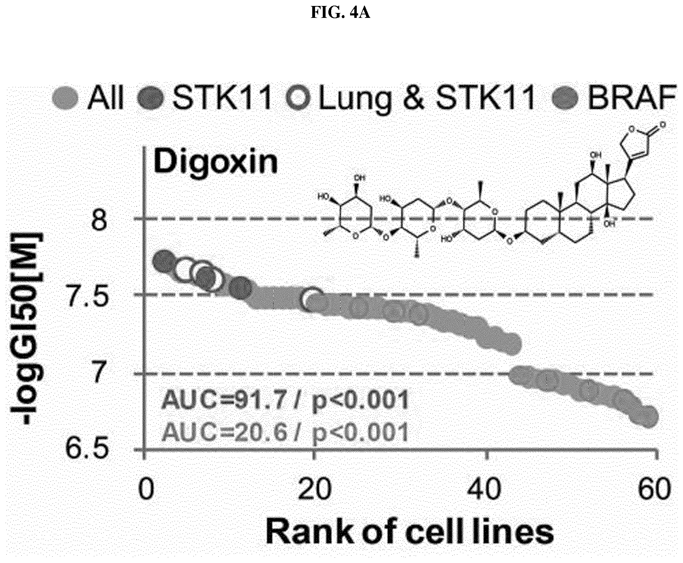

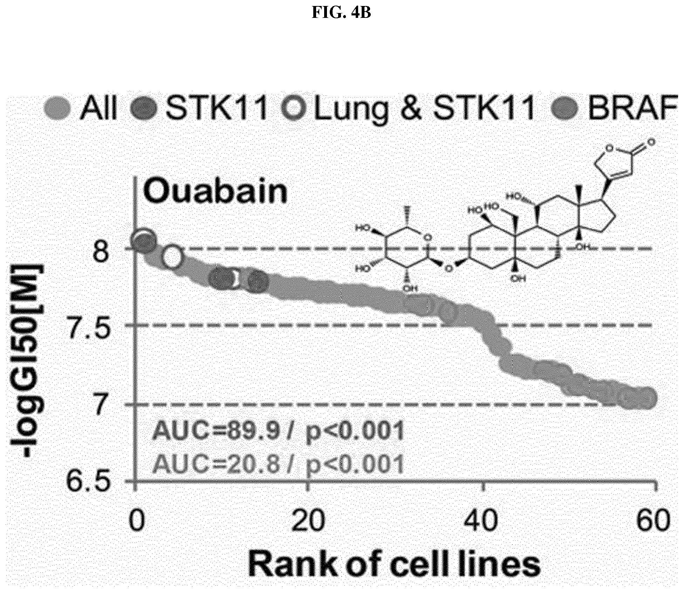

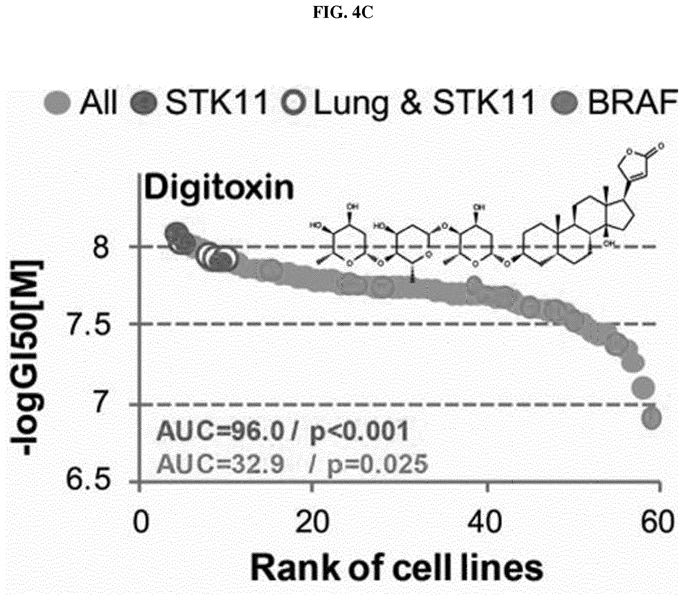

FIG. 4 shows -logGI₅₀ waterfall plots for enrichments of the compounds (digoxin, digitoxin and ouabain) having the respective structures shown in FIG. 1 in STK11 mutant cell lines, compared to those in the wild-type cell line (the plots shown in FIGS. 4A, 4B and 4C to correspond to the compounds, respectively).

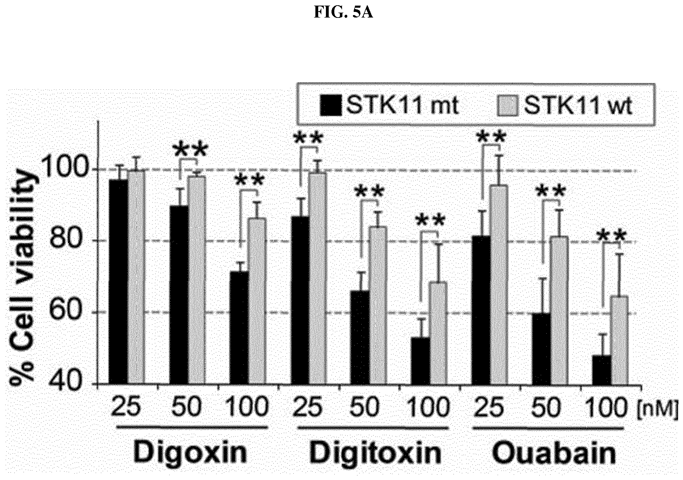

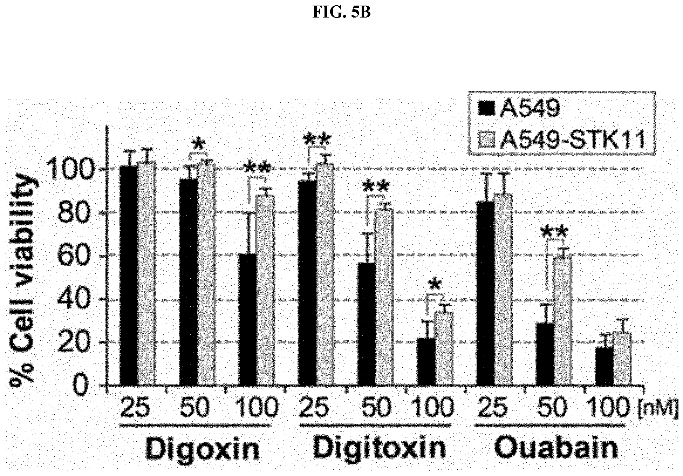

FIG. 5A is a graph of the results of determining inhibition of the cell growth in 8 lung cancer cell lines when a STK11-mutant lung cancer cell line and an STK11-wild-type lung cancer cell line are treated with a varying concentration of digoxin, digitoxin and ouabain (72 hours), and FIG. 5B is a graph of the results of determining inhibition of the cell growth after wild-type STK11 is expressed in the STK11-mutant lung cancer cell line.

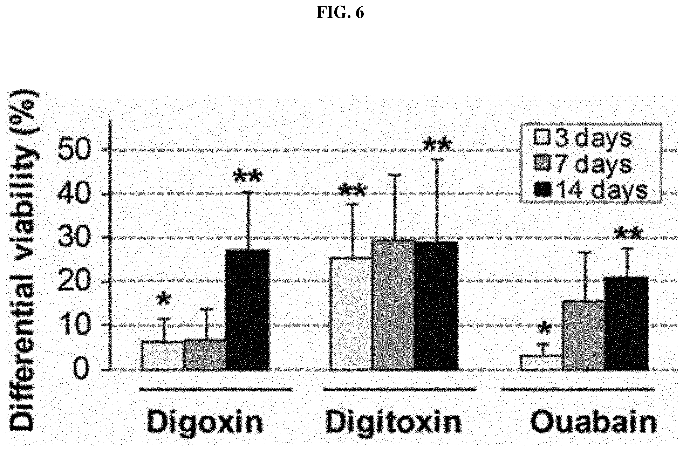

FIG. 6 is a graph of the results of determining effects of digoxin, digitoxin and ouabain on relative inhibition of the cell growth in an STK11 mutant cell line (A549), compared to a cell line (A549-STK11) in which STK11 is normally expressed: the inhibition of the cell growth is determined at the days 3, 7 and 14 (TiterBlue) after the STK11 mutant cell line and the cell line in which STK11 is restored are treated with digoxin, digitoxin and ouabain for a long time.

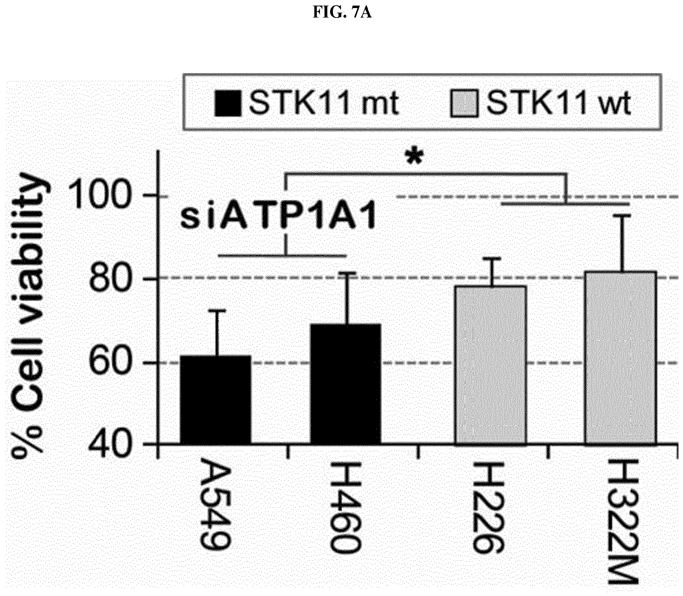

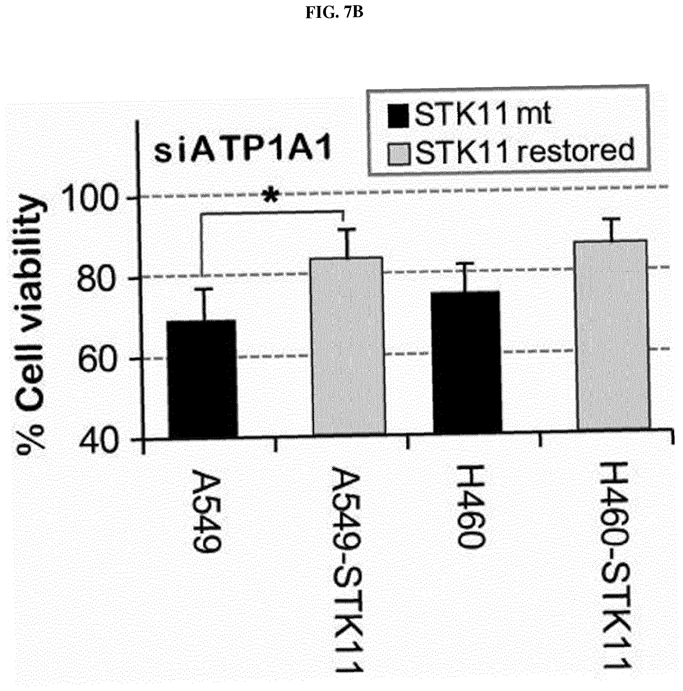

FIGS. 7A and 7B (reference) show results of evaluating cell viability in lung cancer cell lines after lung cancer cell lines are treated with ATP1A1 siRNA (72 hours): FIG. 7A shows TiterBlue results of evaluating cell viability after an STK11-mutant lung cancer cell line and an STK11-wild-type lung cancer cell line are treated with ATP1A1 siRNA, and FIG. 7B shows TiterBlue results of evaluating the cell viability after wild-type STK11 is expressed in the STK11-mutant lung cancer cell line.

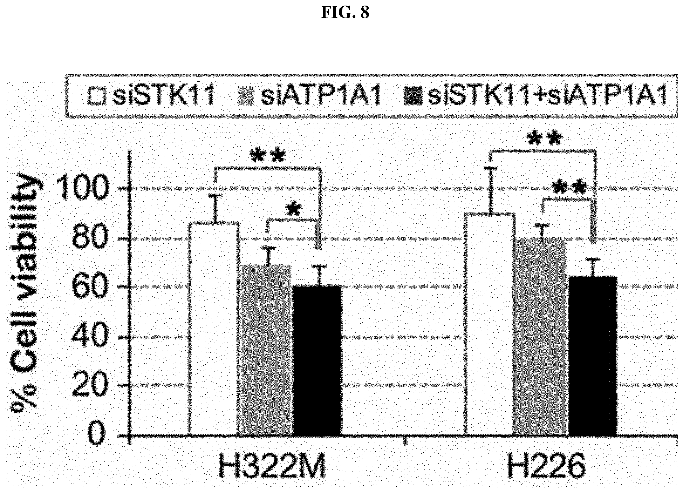

FIG. 8 is a graph of the results of comparing the inhibition of the growth of cells obtained by knocking down both ATP1A1 and STK11 (72 hours) in two lung cancer cell lines having wild-type STK11.

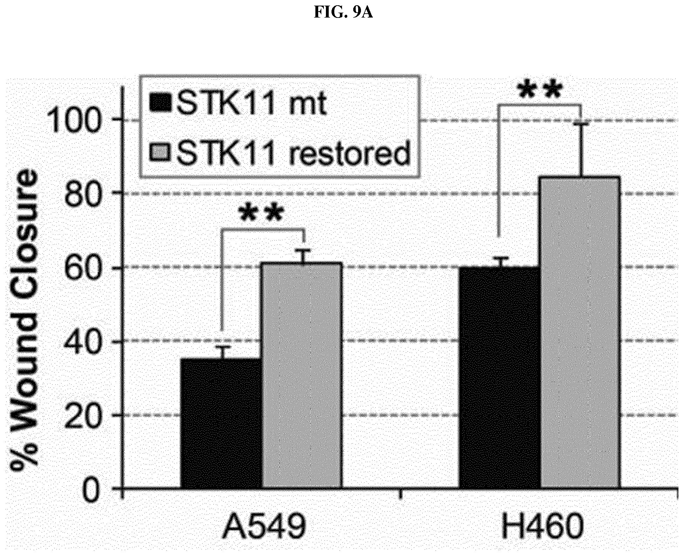

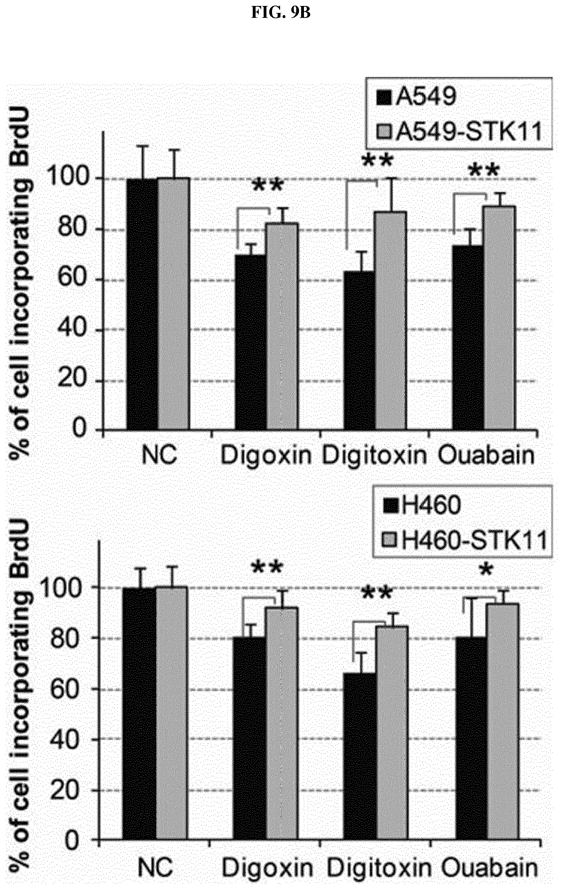

FIG. 9 shows results of determining inhibitory effects on specific cell migration and cell division in STK11-mutant lung cancer cell lines when the STK11-mutant lung cancer cell lines are treated with cardiac glycosides; FIG. 9A shows results of cell migration, and FIG. 9B shows results of cell division.

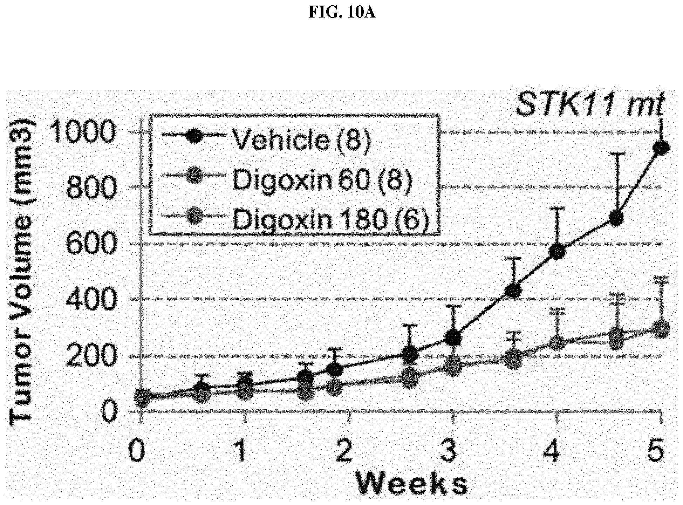

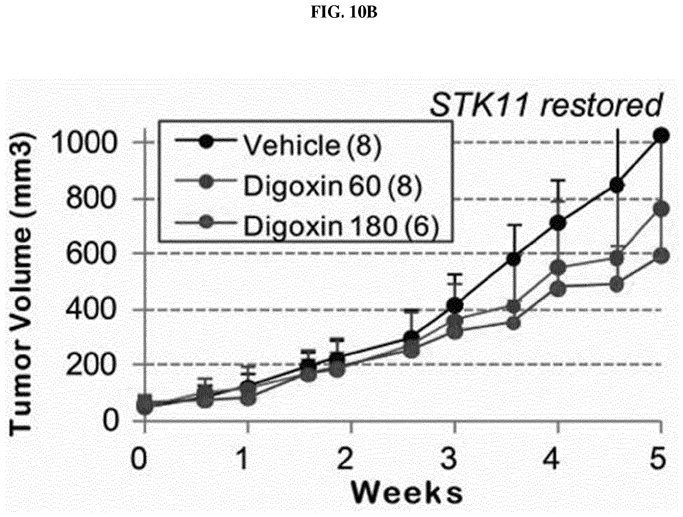

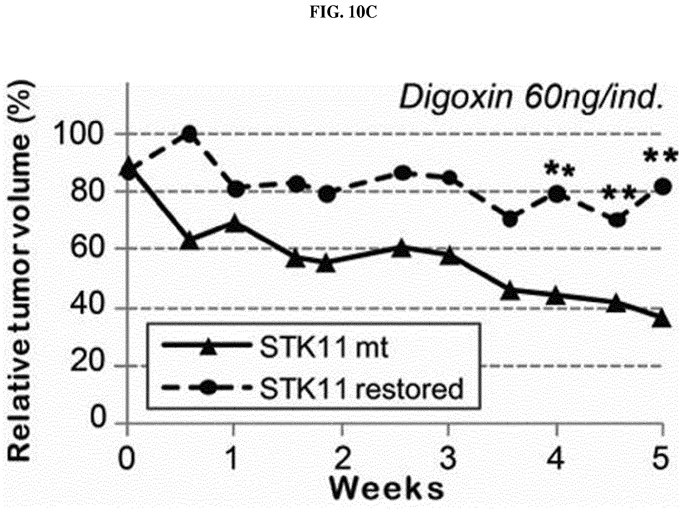

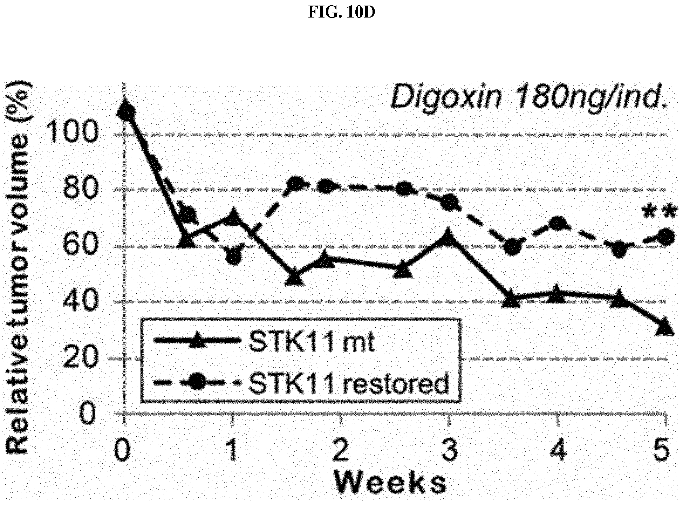

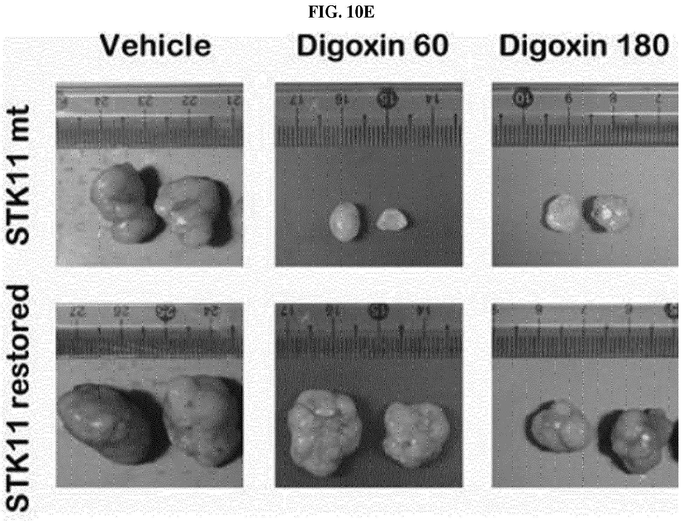

FIG. 10 shows results of determining preclinical effects of cardiac glycosides in STK11-mutant lung cancer: FIG. 10A shows results of determining the growth of a tumor in an animal model into which an A549 cell line is incorporated, FIG. 10B shows results of determining the growth of a tumor in an animal model into which a cell line in which STK11 is restored into the wild type is incorporated, FIGS. 10C, 10D, and 10E are graphs and an image of determining a relative decrease in tumor growth in the animal model into which the STK11-mutant cells are incorporated, compared to that in the animal model into which the cell line in which STK11 is normally expressed is incorporated, when both the cell lines are treated with the cardiac glycosides at a concentration of 60 ng/ind. (3 µg/kg) and 180 ng/ind. (9 µg/kg), respectively.

### [Best Mode]

Hereinafter, the present invention will be described in further detail, as follows.

The present invention is characterized in that it provides a pharmaceutical composition for treating STK11-mutant cancer, which includes, as an active ingredient, a material for inhibiting a sodium-potassium transport function of Na⁺-K⁺ ATPase (ATP1A1), wherein the material comprises a cardiac glycoside selected from the group consisting of digoxin, oubain, and digitoxin.

In the present invention, it has been found by analyzing the characteristics of mutation-specific cancer, which provide new understanding of cancer treatment using various sets of data due to an increase in usefulness of data on human cell lines and tissues that the growth of cancer cells is inhibited when STK11-mutant cancer cells are treated with a material for inhibiting a sodium-potassium transport function of ATP1A1 compared to the wild-type cancer cell line. Therefore, the present invention has been completed based on this fact.

ATP1A1 signaling pathways currently known in the art include activation of mTOR, promotion of a decrease in ATP1A1 activity by AICAR-induced AMPK activation, activation of mitogen-activated protein kinase (MAPK), activation of mitochondrial reactive oxygen species (ROS), activation of phospholipase and inositol triphosphate (IP3) receptors (IP3R), etc.

In the present invention, a material for inhibiting a sodium-potassium transport function of ATP1A1 in STK11-mutant-type cancer, particularly a material specifically binding to ATP1A1 to inhibit an intracellular potassium transport function, is confirmed to be a target for treating STK11-mutation-derived cancer.

Also, according to one exemplary embodiment of the present invention, it was directly confirmed that, when cancer cells are treated with cardiac glycosides as a material for inhibiting a sodium-potassium transport function of ATP1A1, an inhibitory effect on the growth of cancer cells is significantly produced in STK11 mutant-type cancer cells, compared to the wild-type cancer cells (cancer cells having no mutations in a STK11 protein).

In addition, it was experimentally confirmed that, when cancer cells are treated with ATP1A1 siRNA as the material for inhibiting a sodium-potassium transport function of ATP1A1, the ATP1A1 knockdown inhibits the cell growth in STK11-mutated cancer cell lines.

Based on the results, it can be seen that the material for inhibiting a sodium-potassium transport function of ATP1A1 may be used to treat cancers harboring STK11 mutation. This also provides a possible route for therapeutic applications to control the progression of cancer (especially STK11-mutated cancer). It was confirmed that the STK11 knockdown has an effect similar to ATP1A1. The ATP1A1 knockdown induces a selective decrease in the cell growth in STK11 mutant cell lines. This indicates that the expression of ATP1A1 is important for cell growth when normal STK11 signaling disappears. In addition, most ATP1A1 inhibitors have superior cell growth inhibitory activities in STK11 mutant cancer cell lines, compared to the STK11 wild-type cancer cell line.

To sum up, the results of the present invention provide a therapeutic potential for STK11 mutant-type cancer using a novel therapeutic method using the material for inhibiting a sodium-potassium transport function of ATP1A1. Therefore, an approach of the present invention may provide an effective clue to a novel effective therapeutic method.

Therefore, based on the experimental results of the present invention, the present invention may provide a pharmaceutical composition for treating STK11-mutant cancer, which includes, as an active ingredient, a material for inhibiting a sodium-potassium transport function of Na⁺-K⁺ ATPase (ATP1A1), and may also provide a pharmaceutical composition for diagnosing STK11-mutant cancer, which includes, as an active ingredient, a material for inhibiting a sodium-potassium transport function of Na⁺-K⁺ ATPase (ATP1A1), wherein the material comprises a cardiac glycoside selected from the group consisting of digoxin, oubain, and digitoxin.

STK11 mutations are found a wide range of cancers including prostate cancer, breast cancer, colon cancer, lymphoma, melanoma, lung cancer, etc. In this regard, the present inventors have elucidated that a material for inhibiting a sodium-potassium transport function of ATP1A1, particularly a material serving to inhibit an intracellular potassium transport function of ATP1A1 by specifically binding to an ATP1A1 pump to competitively or non-competitively inhibit binding of potassium to ATP1A1, specifically inhibits the growth of STK11-mutant cancer. Most preferably, it has been found that, when the material for inhibiting a sodium-potassium transport function of ATP1A1 is used in lung cancers in which STK11 mutations are detected at higher frequency than that of other cancers, the material for inhibiting a sodium-potassium transport function of ATP1A1 may be effectively applied to treat STK11-mutation lung cancer among the cancers.

An ATP1A1 protein of the present invention may have an amino acid sequence set forth in SEQ ID NO: 1, which is derived from a human, but the present invention is not limited thereto. In this case, the ATP1A1 protein may include a protein having an amino acid sequence having a homology of 70% or more, preferably 80% or more, more preferably 90% or more, and most preferably 95% with the amino acid sequence.

It has been confirmed that the inhibition of ATP1A1 in a series of lung cancer cell lines causes a significant decrease in cell growth in STK11-mutant cancer cell lines other than the wild-type cancer cell line, and it has been found that ATP1A1 plays an important role in cell viability or growth in the STK11-mutant cancer cell lines. These results suggest that ATP1A1 participates in progression of STK11-mutant cancer, indicating that the growth of STK11-mutant cancer cells is inhibited when a function of an ATP1A1 pump is suppressed, which makes it possible to treat STK11-mutant cancer.

In the present invention, the material for inhibiting a sodium-potassium transport function of ATP1A1 is a cardiac glycoside selected from the group consisting of digoxin, oubain, and digitoxin.

The material for inhibiting a sodium-potassium transport function of ATP1A1 is a cardiac glycoside selected from digoxin, ouabain, and digitoxin.

Digoxin binds to a site on an extracellular side of an α-subunit of ATP1A1 (Na⁺-K⁺ ATPase). Ouabain is a compound which is isolated from a plant and widely used in *in vitro* studies by scientists to specifically block a sodium pump. As the cardiac glycoside, digitoxin has a structure and effect similar to digoxin, but the effect of digitoxin lasts for a longer time than digoxin. The above-described digoxin, ouabain, and digitoxin are compounds known to directly and specifically inhibit ATP1A1.

For studies of these currently known compounds, digoxin has been widely used to treat various heart conditions. Digoxin has been proposed to have a beneficial effect on the heart, and reduce the risk of certain carcinoma, but digoxin is just proposed to have an effect of reducing the risk of cancer when the drug is used at a therapeutic concentration.

Considering ouabain, there are known studies showing that ouabain as a cardiac glycoside is a potent therapeutic target against lung cancer, and delays invasion of lung cancer cells, known studies examining that an effect of ouabain on cytotoxicity and a signaling pathway of PC-3 cells in order to determine the potential of the cardiac glycoside to treat androgen-dependent prostate cancers, etc.

Considering digitoxin, there is a known study showing that pharmacological research is performed on digitoxin and analogues thereof to develop a potent chemotherapeutic drug with a focus on the cytotoxicity of the digitoxin and analogues for cancer cells, and a conventional mechanism for an effect of digitoxin on ATP1A1 is established as a new mechanism.

However, there are no studies showing that such cardiac glycosides specifically inhibit the cell growth in STK11-mutant cancer.

The pharmaceutical composition according to the present invention may include the active ingredient at 0.0001 to 50% by weight, based on the total weight of the composition, but the present invention is not limited thereto. The composition according to the present invention may further include a pharmaceutically acceptable carrier for the purpose of use as the pharmaceutical composition. The pharmaceutically acceptable carrier is generally used to prepare a formulation, and includes lactose, dextrose, sucrose, sorbitol, mannitol, starch, gum acacia, calcium phosphate, alginate, gelatin, calcium silicate, microcrystalline cellulose, polyvinyl pyrrolidone, cellulose, water, syrup, methylcellulose, methyl hydroxybenzoate, propyl hydroxybenzoate, talc, magnesium stearate, and mineral oil, but the present invention is not limited thereto. In addition to the above-described components, the pharmaceutical composition of the present invention may further include a lubricant, a wetting agent, a sweetening agent, a flavoring agent, an emulsifying agent, a suspending agent, a preservative, etc. Proper pharmaceutically acceptable carriers and formulations are generally known in the related art.

The pharmaceutical composition of the present invention may be orally or parenterally administered. For parenteral administration, the pharmaceutical composition may be administered by intravenous injection, local injection, intraventricular injection, intrathecal injection, subcutaneus injection, intraperitoneal injection, transdermal administration, etc.

A proper dose of the pharmaceutical composition of the present invention varies, depending on factors including a method of formulation, a mode of administration, age, body weight, sex, and morbidity of a patient, diet, an administration time, a route of administration, an excretion rate, and sensitivity to a reaction. In general, an effective dose of the composition suitable for desired treatment and prevention may be readily determined and prescribed by skilled practitioners. Preferably, the pharmaceutical composition according to the present invention may be administered at a dose of 0.001 to 100 mg/kg/day.

The pharmaceutical composition of the present invention may be prepared into formulations in the form of a unit dose using a pharmaceutically acceptable carrier or excipient, or may be prepared into multi-dose containers according to methods which may be easily performed by those skilled in the art to which the present invention belongs. In this case, the formulation may be in the form of a solution in oil or an aqueous medium, a suspension or an emulsion, or may also be in the form of an extract, a powder, a granule, a tablet, or a capsule, and may further include a dispersing agent or a stabilizing agent.

From the foregoing, the pharmaceutical composition of the present invention may be provided as an anticancer drug for treating STK11 mutant cancer.

Also, the present invention may provide a method for treating STK11 mutant cancer, which includes administering the pharmaceutical composition or the anticancer drug to a subject in need of treatment of STK11 mutant cancer. Here, the subject may include a primate including a human, livestock (cattle, a horse, a sheep, a chicken, a pig, a dog, and a rabbit), etc., but the present invention is not limited thereto.

In the present invention, it was confirmed that the material for inhibiting a sodium-potassium transport function of ATP1A1 inhibits the cell growth in the STK11-mutant cancer cells, compared to the STK11-wild-type cancer cells. Also, it was revealed that the growth of the STK11-mutant cancer cells is inhibited when ATP1A1 is knocked down in the STK11-mutant cancer cells.

Therefore, the material specifically binding to ATP1A1 to inhibit a function of an ATP1A1 pump may inhibit the growth of the STK11-mutant cancer cells to show a cancer treatment effect. In this case, the cancer may be selected from the group consisting of prostate cancer, breast cancer, colon cancer, melanoma, and lung cancer.

### [Mode for Invention]

Hereinafter, the present invention will be described in further detail with reference to examples thereof. However, it should be understood that the following examples provided herein are merely intended to more specifically describe the present invention.

### Example 1: Hierarchical clustering of STK11 genotype-specific compounds

To identify sensitivity patterns of the genotype-specific compounds, a subset of the compounds was selected using a GI₅₀ profile pattern on a CLEA map.

First of all, a -logGI₅₀ value of 8 (that is, GI50 = 0.01 µM) was chosen as a bipartite cutoff used to determine to which cell lines a compound was sensitive on a NCI60 panel. In this case, the compound was sensitive when the value is greater than 8, and was not sensitive when the value is less than 8.

Next, an enrichment score (an AUC value) was used to select the genotype-specific compounds in a CLEA assay.

An AUC value > 80 and a P value < 0.01 were used as cutoff values used to determine that a compound had significant sensitivity to certain genotypes. In this example, compounds showing strong potency (that is, a -logGI₅₀ value of 8) to at least one cell line on the NCI60 panel were included.

As a result, a total of three non-overlapping compounds satisfying the above filtering requirements were compiled. Hierarchical clustering of these selected compounds was carried out on a category of STK11 genotypes. The results are shown in FIG. 3A. In this example, three main groups of compounds showing uniform sensitivity based on the mutant genotypes were identified. Such a cluster of compounds were sensitive to genetic mutations in an STK11 gene. These results provide a direct clue to understanding a mechanism of action (MOA) among the various compounds having similar applications.

Also, FIG. 3B shows that the cardiac glycosides have higher sensitivity to STK11 mutant-type lung cancer, compared to the therapeutic agents for lung cancer (non-small cell lung cancer; NSCLC) previously approved by the FDA.

### Example 2: STK11 genotype-dependent sensitivity of compounds

Three representative compounds (ouabain, digoxin, and digitoxin) were selected from the clusters shown in FIG. 3A to further analyze a genotype-specific cellular reaction.

From the genetic variation pattern shown in FIG. 2A, it was revealed that STK11 (LKB1) mutation is mutually exclusive from other major lung cancer mutations in tissues from a non-small cell lung cancer (NSCLC) patient. Therefore, there was a need for the development of anti-cancer drugs specific to a group of STK11 mutant-type lung cancer patients. From the graph showing the variation frequencies of STK11 mutations according to the progression stage of cancer shown in FIG. 2B, it was confirmed that the frequencies of STK11 mutations increased according to the progression of lung cancer. However, although clinical trials of anti-cancer drugs had been actively conducted on mutations of the genes such as ALK, BRAF, and EGFR in lung cancer, there were no reports on the development of an anticancer drug that targets STK11-mutations with optionally high frequency. Therefore, a need for the development of a pharmaceutical composition having a therapeutic effect specific to STK11-mutant lung cancer is proposed.

Also, STK11 codes for a serine-threonine kinase that directly phosphorylates a central metabolic sensor AMPK to be activated. AMPK regulates lipid, cholesterol, and glucose metabolisms in specialized metabolic tissues such as liver, muscle, and fatty tissue. An STK11 protein participates in two biologically important pathways of causing cancer. First, STK11 aids in maintaining polarized epithelial cells. Second, STK11 activates AMPK controlling the balance of cellular energy. This insight into the functions of STK11 alludes to a proposition that the STK11 may be a target for novel therapeutic strategy through regulation of AMPK activity.

In this example, it was revealed that the ouabain, digoxin, and digitoxin belonged to a cluster associated with the STK11 mutations, and that these compounds showed relatively high sensitivity in 6 cell lines with STK11 mutation potential, as shown in FIG. 3A.

The 6 cell lines with STK11 mutation potential are listed in Table 1 below.

**[Table 1]**

| MOLT-4 | Leukemia | CDKN2A; NOTCH1; NRAS; PTEN; TP53; STK11; |
|---|---|---|
| A549 | **Lung** | CDKN2A; KRAS; STK11 |
| NCI-H460 | **Lung** | CDKN2A; KRAS; PIK3CA; STK11; c-MYC-Amp |
| NCI-H23 | **Lung** | KRAS; STK11; TP53; PTEN; CTNNB1 |
| SK-MEL-5 | Melanoma | BRAF; CDKN2A; STK11 |
| DU-145 | Prostate | CDKN2A; MLH1; RB1; STK11; TP53 |

Meanwhile, FIG. 4 shows -logGI₅₀ waterfall plots for enrichments of the compounds (digoxin, digitoxin, and ouabain) having the respective structures shown in FIG. 1 in STK11 mutant cell lines, compared to those in the wild-type cell line (the plots shown in FIGS. 4A, 4B and 4C to correspond to the compounds, respectively).

From the results shown in FIGS. 3 and 4, it was revealed that all the three cardiac glycoside drugs clearly showed the STK11-specific sensitivity.

As a result, it can be defined that the cardiac glycosides drugs had STK11 mutation-dependent activity.

### Example 3: Analysis of inhibitory effect specific to STK11-mutant lung cancers on cell growth by treatment of cardiac glycosides

### (1) Cell line culture

NCI-60 lung cancer cell lines (NCI-H460, A549, NCI-H322M, and NCI-H226) were obtained from the U.S. National Cancer Institute (NCI DTP). All cells were grown in an RPMI medium (GIBCO) supplemented with 10% FBS (GIBCO) and penicillin/streptavidin (GIBCO), and kept at 37°C in a humidified atmosphere of 5% CO₂.

### (2) Analysis of cell viability

A total of three cardiac glycosides (chemicals) having the structures shown in FIG. 1 were screened in a lung cancer cell line. Digoxin, ouabain, and digitoxin were all purchased from Sigma Aldrich.

The cells were seeded in 96-well plates at a density of 2,000, 1,000, and 200 cells per well, and cultured for 3, 7, and 14 days, respectively. After 24 hours of seeding, the cells were treated with a chemical diluted with digitoxin and ouabain at working concentrations of 0.05 µM and 0.02 µM, respectively. The cells were again cultured for 72 hours (3 days), 7 days, and 14 days, and then the cell viability was measured using a CellTiter-Blue assay kit.

Four STK11-mutant lung cancer cell lines (A549, H460, H1993, and H1395) and four STK11-wild-type lung cancer cell lines (H226, H322M, H82, and H524) were treated with a varying concentration of digoxin, digitoxin, and ouabain as the cardiac glycosides for 72 hours to check whether the cell growth was inhibited. The respective results are shown in FIG. 5A.

From the results of FIG. 5A, it can be seen that the STK11-mutant lung cancer cell lines had higher sensitivity to the respective compounds, compared to the STK11-wild-type lung cancer cell lines.

Therefore, it was confirmed that the cell growth was inhibited when the lung cancer cell lines were treated with digoxin, digitoxin, and ouabain in the 96-well plate. In this case, each of a STK11-mutant cell line (A549) and a cell line (A549-STK11) in which STK11 was restored into the wild type were used as the lung cancer cell line.

FIG. 5B is a graph of the results of determining inhibition of the cell growth after the cell lines are treated with each of digoxin, digitoxin, and ouabain (TiterBlue). It can be seen that the cell line in which STK11 was restored was resistant to the treated compounds, compared to the STK11-mutant cell line.

Also, FIG. 6 shows a difference in viability between the STK11-mutant cell line (A549) and the cell line (A549-STK11) in which STK11 was restored into the wild type when both of the cell lines are treated with the cardiac glycosides for a long time.

As shown in FIG. 6, it was revealed that a growth inhibitory effect on the mutant-type cell line tended to increase, compared to the cell line in which STK11 was restored into the wild type, when both of the cell lines were treated with the cardiac glycosides for a long time (3 days, 7 days, and 14 days). Therefore, ATP1A1 inhibition using effective drugs may provide an effective treatment of the patient's tumor containing STK11 mutations.

These results showed that digoxin, digitoxin, or ouabain specifically inhibited the cell growth in the STK11 mutation cancer.

### Example 4: Analysis of inhibitory effect specific to STK11-mutant lung cancer on cell growth by ATP1A1 knockdown

### (1) siRNA transfection

For siRNA transfection, the cells were plated on a 96-well plate at a density of 3,000 cells/well. After the cells were attached for 24 hours, target siRNAs (ATP1A1: 1009769 (Bioneer); STK11: L-005035-00; and Non-targeting: D-001810-10 (Dharmacon)) were added to a transfection medium (GIBCO) at 37 °C for 6 hours in a CO₂ incubator. After the transfection, an RPMI medium including FBS was further added to the cells, and the cells were then collected and cultured at 37 °C, with CO₂ for 72 hours in an incubator.

### (2) Analysis of correlation between STK11 mutations and ATP1A1 expression in lung cancer

FIG. 7A shows TiterBlue results of evaluating cell viability obtained after four lung cancer cell lines are treated with ATP1A1 siRNA (for 72 hours).

From the results of FIG. 7A, it can be seen that the growth of the STK11-mutant lung cancer cell lines A549 and H460 were significantly inhibited, compared to the STK11-wild-type lung cancer cell lines H226 and H322, when the lung cancer cell lines were treated with the ATP1A1 siRNA. That is, when ATP1A1 was knocked down in a series of lung cancer cell lines, the cell growth was significantly reduced in the STK11-mutant cell lines rather than the STK11-wild-type cell lines. Also, FIG. 7B shows TiterBlue results of expressing wild-type STK11 in the STK11-mutant lung cancer cell lines A549 and H460. It can be seen that the cell growth was less reduced due to the ATP1A1 knockdown.

Therefore, ATP1A1 seems to play an important role in cancer survival or growth in the STK11-mutated lung cancer cell lines.

Next, FIG. 8 shows results of inhibiting the cell growth obtained by knocking down both ATP1A1 and STK11 (72 hours) in two lung cancer cell lines having wild-type STK11. It can be seen that the cell growth was synergistically inhibited in the STK11-wild-type lung cancer cell lines when both of ATP1A1 and STK11 were knocked down, compared to when either ATP1A1 or STK11 was knocked down.

### Example 5: Inhibitory effect of cardiac glycosides on cell migration and division specific to STK11 mutations

The STK11-mutant lung cancer cell lines A549 and H460 (STK11 mutants) and the cell lines A549-STK11 and H460-STK11 in which STK11 was restored into the wild type (STK11 restored) were treated with the cardiac glycoside (digoxin, digitoxin, or ouabain) to evaluate an effect on cell migration and cell division.

For a cell migration assay, a Transwell cell culture chamber (Costar 3422, Cambridge, MA) equipped with a filter having a pore size of 12 µm was used. More specifically, a cell suspension (5×10⁴ cells/500 µl of a culture medium) was added to the top of the filter, and treated with each of the cardiac glycosides. After 24 hours, the cells migrating to the bottom of the filter were counted in 10 random fields using a phase-contrast microscope with 100× magnification. Also, a BrdU assay was used as the cell division assay. More specifically, the cells were inoculated in a 96-well plate at a density of 2,500 cells/well, treated with the cardiac glycoside (100 nM digoxin, 50 nM digitoxin, or 50 nM ouabain) after 24 hours, and then cultured again for 24 hours. Thereafter, the cells were labeled with bromodeoxyuridine (BrdU) for 2 hours. Then, the assay was performed by detecting the labeled cells using an ELISA reader (Cell Proliferation ELISA, BrdU (colorimetric), Roche).

As a result, as seen from the analysis results of the cell migration of FIG. 9A and the cell division of FIG. 9B, it can be seen that the cell migration and division were significantly decreasedwhen the STK11-mutant lung cancer cell lines were treated with the cardiac glycosides. The results indicate that the cardiac glycosides inhibited the migration and division of the STK11-mutant lung cancer cells.

### Example 6: STK11-mutation-specific anticancer effects of cardiac glycosides in animal model

To examine whether an ATP1A1 inhibitor had a preclinical effect on STK11-mutant lung cancer, this experiment was performed using the animal model in which an A549 lung cancer cell line or a cell line (A549-LKB1wt) in which STK11 was restored into the wild type was transplanted into mice (a mouse tumor xenograft model). After athymic nude mice (Oriental Bio Inc) were purchased, the animal model was prepared according to the standard guidelines provided by the Catholic University of Korea-Institutional Animal Care and Use Committee (CUK-IACUC). A549 or A549-LKBlwt were subcutaneously injected (3×10⁶ cells/ml) into the above animal model, and divided into three groups: (1) an untreated control group (n = 10); (2) a group in which the mice were treated with digoxin at 60 ng/kg/body weight (n = 10); (3) a group in which the mice were treated with digoxin at 180 ng/kg/body weight (n = 10). Digoxin or a vehicle (1.9% DMSO/10% polyethylene glycol 400/1× PBS: a total volume of 200 µl) was orally administered three times a week for 5 weeks. Then, the diameter of each tumor base was measured, and the size of a tumor was then calculated according to the following equation: Tumor size (mm³) = (length × width × height × 0.52). Also, the mice were monitored until the tumor size reached 1 cm³, and then the mice were sacrificed, and tumors were extracted from the mice.

FIG. 10A shows that the growth of a tumor in the animal model in which the A549 cell line was injected into the mice was reduced in a time-dependent manner when the cardiac glycoside (i.e., digoxin) was orally administered to the mice, and FIG. 10B shows results of determining that the cardiac glycoside did not have the same effect as described above in the animal model in which the cell line in which STK11 was restored into the wild type was injected into the mice.

Therefore, changes in the size of a tumor in the STK11 mutant types and the cell line in which STK11 was restored (wild-type STK11) were compared and analyzed. As shown in FIGS. 10C, 10D, and 10E, it can be seen that the tumor growth was selectively reduced by treatment with digoxin in the animal model in which the STK11-mutant cells were injected into the mice, compared to the animal model in which the wild-type STK11-expressed cells were injected into the mice. Interestingly, it was revealed that the selective sensitivities to the cardiac glycosides were significant when the cardiac glycosides was administered at a low drug dose [60 ng/ind. (3 µg/kg)], compared to when the cardiac glycosides was administered at a high drug dose [180 ng/ind. (9 µg/kg)]. While the concentration of the prescribed digoxin used in the conventional clinical trials was 250 mcg/day on average in the case of adults, it can be seen that an expected concentration of the cardiac glycoside to be prescribed for STK11-mutant-type lung cancer would be very low at 180 mcg/day or less on average in the case of adults.

The results indicate that the cardiac glycosides had a preclinical anti-cancer effect specific to the STK11-mutant lung cancer.

The present invention has been described in detail. However, it should be understood that the detailed description and specific examples, while indicating preferred embodiments of the invention, are given by way of illustration only, since various changes and modifications within the spirit and scope of the invention will become apparent to those skilled in the art from this detailed description.

### [Industrial Applicability]

The present invention can be applied to the treatment of STK11-mutant lung cancer.
## Claims

1. A pharmaceutical composition for use in treating cancers harboring serine/threonine kinase 11 (STK11) mutation, comprising, as an active ingredient, a material for inhibiting a sodium-potassium transport function of sodium/potassium-transporting ATPase subunit alpha-1 (Na⁺-K⁺ ATPase subunit alpha-1, ATP1A1), wherein the material comprises a cardiac glycoside selected from the group consisting of digoxin, ouabain, and digitoxin.

2. The pharmaceutical composition for use of claim 1, wherein the cancer is selected from the group consisting of prostate cancer, breast cancer, colon cancer, melanoma, and lung cancer.

## Patentansprüche

1. Pharmazeutische Zusammensetzung zur Verwendung bei der Behandlung von Krebserkrankungen, die eine Mutation von Serin/Threonin-Kinase 11 (STK11) beherbergen, die als Wirkstoff ein Material zum Inhibieren einer Natrium-Kalium-Transportfunktion einer Natrium/Kalium transportierenden ATPase-Untereinheit alpha-1 (Na⁺-K⁺-ATPase-Untereinheit alpha-1, ATP1A1) umfasst, wobei das Material ein Herzglykosid umfasst, das aus der Gruppe ausgewählt ist, die aus Digoxin, Ouabain und Digitoxin besteht.

2. Pharmazeutische Zusammensetzung zur Verwendung nach Anspruch 1, wobei der Krebs aus der Gruppe ausgewählt ist, die aus Prostatakrebs, Brustkrebs, Darmkrebs, Melanom und Lungenkrebs besteht.

## Revendications

1. Composition pharmaceutique pour une utilisation dans le traitement de cancers porteurs de la mutation de la sérine/thréonine kinase 11 (STK11), comportant, en tant qu'ingrédient actif, une substance destinée à inhiber une fonction de transport de sodium-potassium de la sous-unité alpha-1 de l'ATPase de transport de sodium/potassium (sous-unité alpha-1 de l'ATPase Na⁺-K⁺, ATP1A1), dans laquelle la substance comporte un glycoside cardiaque choisi parmi le groupe constitué de la digoxine, l'ouabaïne et la digitoxine.

2. Composition pharmaceutique pour une utilisation selon la revendication 1, dans laquelle le cancer est choisi parmi le groupe constitué d'un cancer de la prostate, d'un cancer du sein, d'un cancer du côlon, d'un mélanome et d'un cancer du poumon.


<110> Industry-Academic Cooperation Foundation, Sookmyung Women's University
<120> PHARMACEUTICAL COMPOSITIONS FOR TREATMENT OF STK11-MUTATED CANCER COMPRISING CARDIAC GLYCOSIDES
<130> PCTB02350
<150> KR 10-2014-0032585
   <151> 2014-03-20
<150> KR 10-2015-0038485
   <151> 2015-03-19
<160> 1
<170> KopatentIn 2.0
<210> 1
   <211> 1023
   <212> PRT
   <213> Homo sapiens / sodium-potassium transporting ATPase subunit alpha 1
   isoform
<400> 1
   Met Gly Lys Gly Val Gly Arg Asp Lys Tyr Glu Pro Ala Ala Val Ser
    1               5                  10                  15
   Glu Gln Gly Asp Lys Lys Gly Lys Lys Gly Lys Lys Asp Arg Asp Met
               20                  25                  30
   Asp Glu Leu Lys Lys Glu Val Ser Met Asp Asp His Lys Leu Ser Leu
           35                  40                  45
   Asp Glu Leu His Arg Lys Tyr Gly Thr Asp Leu Ser Arg Gly Leu Thr
       50                  55                  60
   Ser Ala Arg Ala Ala Glu Ile Leu Ala Arg Asp Gly Pro Asn Ala Leu
   65                  70                  75                  80
   Thr Pro Pro Pro Thr Thr Pro Glu Trp Ile Lys Phe Cys Arg Gln Leu
                   85                  90                  95
   Phe Gly Gly Phe Ser Met Leu Leu Trp Ile Gly Ala Ile Leu Cys Phe
               100                 105                 110
   Leu Ala Tyr Ser Ile Gln Ala Ala Thr Glu Glu Glu Pro Gln Asn Asp
           115                 120                 125
   Asn Leu Tyr Leu Gly Val Val Leu Ser Ala Val Val Ile Ile Thr Gly
       130                 135                 140
   Cys Phe Ser Tyr Tyr Gln Glu Ala Lys Ser Ser Lys Ile Met Glu Ser
   145                 150                 155                 160
   Phe Lys Asn Met Val Pro Gln Gln Ala Leu Val Ile Arg Asn Gly Glu
                   165                 170                 175
   Lys Met Ser Ile Asn Ala Glu Glu Val Val Val Gly Asp Leu Val Glu
               180                 185                 190
   Val Lys Gly Gly Asp Arg Ile Pro Ala Asp Leu Arg Ile Ile Ser Ala
           195                 200                 205
   Asn Gly Cys Lys Val Asp Asn Ser Ser Leu Thr Gly Glu Ser Glu Pro
       210                 215                 220
   Gln Thr Arg Ser Pro Asp Phe Thr Asn Glu Asn Pro Leu Glu Thr Arg
   225                 230                 235                 240
   Asn Ile Ala Phe Phe Ser Thr Asn Cys Val Glu Gly Thr Ala Arg Gly
                   245                 250                 255
   Ile Val Val Tyr Thr Gly Asp Arg Thr Val Met Gly Arg Ile Ala Thr
               260                 265                 270
   Leu Ala Ser Gly Leu Glu Gly Gly Gln Thr Pro Ile Ala Ala Glu Ile
           275                 280                 285
   Glu His Phe Ile His Ile Ile Thr Gly Val Ala Val Phe Leu Gly Val
       290                 295                 300
   Ser Phe Phe Ile Leu Ser Leu Ile Leu Glu Tyr Thr Trp Leu Glu Ala
   305                 310                 315                 320
   Val Ile Phe Leu Ile Gly Ile Ile Val Ala Asn Val Pro Glu Gly Leu
                   325                 330                 335
   Leu Ala Thr Val Thr Val Cys Leu Thr Leu Thr Ala Lys Arg Met Ala
               340                 345                 350
   Arg Lys Asn Cys Leu Val Lys Asn Leu Glu Ala Val Glu Thr Leu Gly
           355                 360                 365
   Ser Thr Ser Thr Ile Cys Ser Asp Lys Thr Gly Thr Leu Thr Gln Asn
       370                 375                 380
   Arg Met Thr Val Ala His Met Trp Phe Asp Asn Gln Ile His Glu Ala
   385                 390                 395                 400
   Asp Thr Thr Glu Asn Gln Ser Gly Val Ser Phe Asp Lys Thr Ser Ala
                   405                 410                 415
   Thr Trp Leu Ala Leu Ser Arg Ile Ala Gly Leu Cys Asn Arg Ala Val
               420                 425                 430
   Phe Gln Ala Asn Gln Glu Asn Leu Pro Ile Leu Lys Arg Ala Val Ala
           435                 440                 445
   Gly Asp Ala Ser Glu Ser Ala Leu Leu Lys Cys Ile Glu Leu Cys Cys
       450                 455                 460
   Gly Ser Val Lys Glu Met Arg Glu Arg Tyr Ala Lys Ile Val Glu Ile
   465                 470                 475                 480
   Pro Phe Asn Ser Thr Asn Lys Tyr Gln Leu Ser Ile His Lys Asn Pro
                   485                 490                 495
   Asn Thr Ser Glu Pro Gln His Leu Leu Val Met Lys Gly Ala Pro Glu
               500                 505                 510
   Arg Ile Leu Asp Arg Cys Ser Ser Ile Leu Leu His Gly Lys Glu Gln
           515                 520                 525
   Pro Leu Asp Glu Glu Leu Lys Asp Ala Phe Gln Asn Ala Tyr Leu Glu
       530                 535                 540
   Leu Gly Gly Leu Gly Glu Arg Val Leu Gly Phe Cys His Leu Phe Leu
   545 550 555 560
   Pro Asp Glu Gln Phe Pro Glu Gly Phe Gln Phe Asp Thr Asp Asp Val
   565 570 575
   Asn Phe Pro Ile Asp Asn Leu Cys Phe Val Gly Leu Ile Ser Met Ile
   580 585 590
   Asp Pro Pro Arg Ala Ala Val Pro Asp Ala Val Gly Lys Cys Arg Ser
   595 600 605
   Ala Gly Ile Lys Val Ile Met Val Thr Gly Asp His Pro Ile Thr Ala
   610 615 620
   Lys Ala Ile Ala Lys Gly Val Gly Ile Ile Ser Glu Gly Asn Glu Thr
   625 630 635 640
   Val Glu Asp Ile Ala Ala Arg Leu Asn Ile Pro Val Ser Gln Val Asn
   645 650 655
   Pro Arg Asp Ala Lys Ala Cys Val Val His Gly Ser Asp Leu Lys Asp
   660 665 670
   Met Thr Ser Glu Gln Leu Asp Asp Ile Leu Lys Tyr His Thr Glu Ile
   675 680 685
   Val Phe Ala Arg Thr Ser Pro Gln Gln Lys Leu Ile Ile Val Glu Gly
   690 695 700
   Cys Gln Arg Gln Gly Ala Ile Val Ala Val Thr Gly Asp Gly Val Asn
   705 710 715 720
   Asp Ser Pro Ala Leu Lys Lys Ala Asp Ile Gly Val Ala Met Gly Ile
   725 730 735
   Ala Gly Ser Asp Val Ser Lys Gln Ala Ala Asp Met Ile Leu Leu Asp
   740 745 750
   Asp Asn Phe Ala Ser Ile Val Thr Gly Val Glu Glu Gly Arg Leu Ile
   755 760 765
   Phe Asp Asn Leu Lys Lys Ser Ile Ala Tyr Thr Leu Thr Ser Asn Ile
   770 775 780
   Pro Glu Ile Thr Pro Phe Leu Ile Phe Ile Ile Ala Asn Ile Pro Leu
   785 790 795 800
   Pro Leu Gly Thr Val Thr Ile Leu Cys Ile Asp Leu Gly Thr Asp Met
   805 810 815
   Val Pro Ala Ile Ser Leu Ala Tyr Glu Gln Ala Glu Ser Asp Ile Met
   820 825 830
   Lys Arg Gln Pro Arg Asn Pro Lys Thr Asp Lys Leu Val Asn Glu Arg
   835 840 845
   Leu Ile Ser Met Ala Tyr Gly Gln Ile Gly Met Ile Gln Ala Leu Gly
   850 855 860
   Gly Phe Phe Thr Tyr Phe Val Ile Leu Ala Glu Asn Gly Phe Leu Pro
   865 870 875 880
   Ile His Leu Leu Gly Leu Arg Val Asp Trp Asp Asp Arg Trp Ile Asn
                   885                 890                 895
   Asp Val Glu Asp Ser Tyr Gly Gln Gln Trp Thr Tyr Glu Gln Arg Lys
               900                 905                 910
   Ile Val Glu Phe Thr Cys His Thr Ala Phe Phe Val Ser Ile Val Val
           915                 920                 925
   Val Gln Trp Ala Asp Leu Val Ile Cys Lys Thr Arg Arg Asn Ser Val
       930                 935                 940
   Phe Gln Gln Gly Met Lys Asn Lys Ile Leu Ile Phe Gly Leu Phe Glu
   945                 950                 955                 960
   Glu Thr Ala Leu Ala Ala Phe Leu Ser Tyr Cys Pro Gly Met Gly Val
                   965                 970                 975
   Ala Leu Arg Met Tyr Pro Leu Lys Pro Thr Trp Trp Phe Cys Ala Phe
               980                 985                 990
   Pro Tyr Ser Leu Leu Ile Phe Val Tyr Asp Glu Val Arg Lys Leu Ile
           995                 1000                1005
   Ile Arg Arg Arg Pro Gly Gly Trp Val Glu Lys Glu Thr Tyr Tyr
      1010                1015                1020